# EUROPEAN PATENT APPLICATION

(11) **EP 1 738 688 A2**
(43) Date of publication of application: **03.01.2007**
(21) Application number: 06253364.1
(22) Date of filing: 28.06.2006
(51) Int. Cl.: A61B 5/103

(54) **Apparatus & method for viewing the skin**

(30) Priority: 29.06.2005 US 169942
(71) Applicant: JOHNSON & JOHNSON CONSUMER COMPANIES, INC., Skillman, NJ 08558 (US)
(72) Inventor: Menke, James, Califon, NJ 07830 (US); Cole, Curtis A., Ringoes, NJ 08551 (US); Pien, Katty, Rancho Palos Verdes California 9075 (US)
(74) Representative: Tunstall, Christopher Stephen

(57) **Abstract**

An apparatus and method for aiding a person to conduct self-examination of the skin in a mirror has a light source emanating light that impinges upon the skin of the person, causing light to be reflected from the skin and causing the skin to emit light, the reflected and emitted light impinging on the mirror and further reflecting from the mirror to the eye of the person. A pair of goggles worn by the person has lenses that are light filters and/or polarizers. The light source may be in the form of a flashlight which has filters and/or polarizers disposed over the output. The lenses and polarizer are preferably removable and the polarizer may be rotatable to provide various illuminating light combinations. The image reviewed by the person as a consequence of the lenses in the goggles and over the flashlight are enhanced for viewing features of the skin depending upon the combination of lenses, filters, polarizers and their respective relative orientations.

## Description

### Field of the Invention

The present invention relates to apparatus and method for viewing the skin, and more particularly, for viewing the skin under different wavelengths and polarization of light.

### Background of the Invention

The monitoring and maintenance of healthy skin is an important concern for most people. Typically people examine their skin using a mirror in a setting with natural, incandescent and/or fluorescent lighting. This self examination process is used by a person to ascertain the condition of their skin and potentially to treat the skin with various therapies and preparations in order to improve the condition of the skin. For example, upon viewing the skin in the mirror and ascertaining that the skin looks oily, the selection and use of a washing and/or drying agent may be employed. The presence of wrinkled skin may indicate that a moisturizer or other wrinkle treatment would be advisable. People with acne frequently check their skin in the mirror to monitor and treat the acne condition. In addition to the skin conditions that are readily visible in normal lighting environments, there are also conditions that are invisible to inspection using a mirror in typical lighting. For example, conditions of the skin, such as the dilation of blood vessels below the surface, and UV photo damage to subsurface layers (mainly due to exposure to the sun), etc., will not necessarily be apparent by simply viewing the surface of the skin in a mirror. It is now known that inspection of the skin utilizing various wavelengths of light and/or polarized light can illuminate and reveal skin conditions which would otherwise be imperceptible. In addition, these alternative illuminating techniques can highlight and emphasize visible conditions, such as wrinkles or acne. Known techniques for sub-surface or enhanced surface viewing typically involve photography, wherein a flash unit which is capable of producing light of a particular wavelength is activated and an image captured with a camera. Various filters may also be employed in this process. For example, polarized photography has been utilized to enhance the surface or subsurface features of the skin by placing the polarizer in front of a flash unit and in front of a camera, and a photograph of the skin taken under these conditions. When the pictures obtained are examined, surface features of the skin, such as scales, wrinkles, fine lines, pores, and hairs are visually enhanced. When the polarizers are arranged perpendicular to each other, sub-surface features of the skin such as erythema pigmentation and blood vessels are visually enhanced. When the polarizers are in the same orientation, surface features of the skin such as scales, wrinkles, fine lines, pores and hairs are visually enhanced. Ultraviolet (UV) photography utilizing a flash unit filtered to produce ultraviolet A light and a camera is filtered so that only visible light enters the lens produces images that are visually enhanced with regard to pigmentation, the presence of the bacteria p. acnes and horn. A variation of ultraviolet photography has been termed the "sun camera" where ultraviolet A light is used to illuminate the skin and an ultraviolet A sensitive digital camera is used to record the ultraviolet light reflected from the skin. In this arrangement, both pigment distribution and the surface features of the skin are visually enhanced. While the foregoing photographic techniques have proven valuable and useful for analyzing the condition of the skin, they require fairly sophisticated and expensive equipment and the use of photographic techniques. There is a need therefore for an inexpensive and uncomplicated apparatus and method for enhanced visualization of the skin.

### Summary of the Invention

The problems and disadvantages associated with conventional apparatus and techniques utilized to view the skin are overcome by the present invention, which includes apparatus and methods for aiding a person illuminated by a light source to view their skin in a mirror. The light source emanates light that impinges upon the skin of the person, causing light to be reflected from the skin and causing the skin to emit light, the reflected and emitted light impinging on the mirror and further reflecting from the mirror to the eye of the person. The emanation of light from the light source and subsequent reflections and emanation from the skin and the mirror to the eye define a path of light energy from the light source to the eye. A light modification element other than a magnifying lens is interposed in the path of light energy between the light source and the eye of the person viewing the skin, the light modification element enhancing the visualization of at least one attribute of the skin. In accordance with a method for conducting self-examination of the skin in a mirror, a person illuminates the skin with a light source emanating light of a selected wavelength and state of polarization/nonpolarization to create a path of light energy from the light source to the eye of the person. A light modification element other than a magnifying lens is positioned in the path of light to enhance the person's view of themselves in the mirror, such that the perceptibility of an attribute of their skin is visually enhanced over that otherwise viewable in the mirror without the light modification element.

### Brief Description of the Drawings

FIG. 1 is a perspective view of a person examining their skin in a mirror and using an embodiment of the present invention;
FIG. 2 is a diagrammatic view of the invention of FIG. 1;
FIG. 3 is another diagrammatic view of the invention of FIG. 1 showing different combinations of illuminating light and filtering for viewing surfaces S₁ through S₄;
FIG. 4 is a front view of the light source shown in FIGS. 1-3;
FIG. 5 is a perspective view of a person using a second embodiment of the present invention;
FIG. 6 is a diagrammatic view of a cross section of the invention shown in FIG. 5 taken along section lines VI - VI and looking in the direction of the arrows; and
FIG. 7 is a front view of the invention shown in FIGS. 5 and 6.

### Detailed Description of the Invention

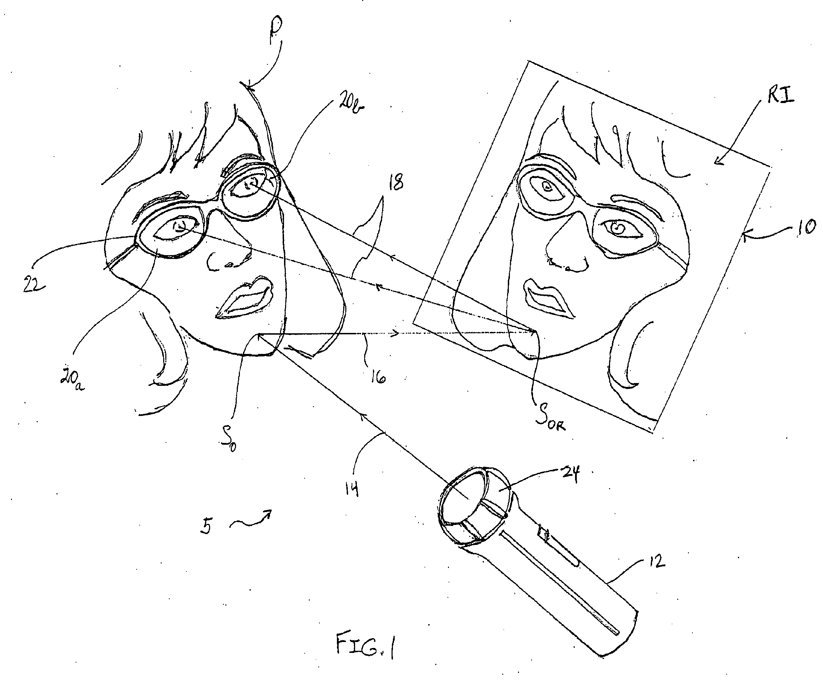

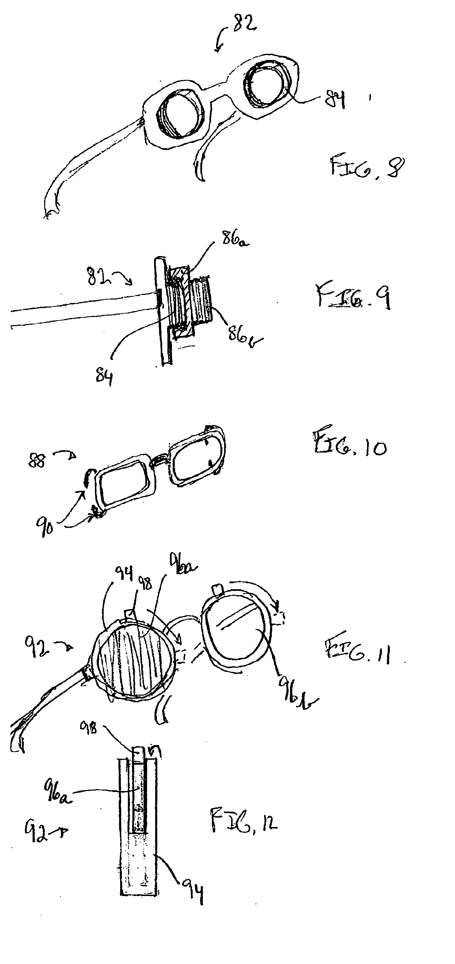

FIG. 1 shows a visualizing system 5 used by a person P examining her reflected facial image RI in a mirror 10. Light source 12 illuminates the face of the person P, for example, at surface S₀ via illuminating light 14. The illuminating light 14 reflects off the surface S₀ and/or causes the skin or other surface components of the surface S₀ as well as the subsurface layers of the skin proximate to S₀ to fluoresce. The resultant reflected and or fluorescent light 16 emanates from the surface S₀ and impinges on corresponding reflected surface Sₒᵣ on the mirror image RI. Light 16 is reflected from the mirror 10 as indicated by lines 18 and passes through lenses 20ₐ, 20_{b} of goggles/eyeglasses 22 where it enters the eye of the person P to allow examination of So by that person. The light source 12 may have a polarizer and/or filter portion 24 which may be rotated relative to light source 12 and/or removed depending on the imaging that is desired. In figure 2, the lens 20ₐ, of the goggles 22 is shown as being rotatable, for example if it is a polarizer. A polarizer and/or filter lens 24 may be interposed at the output of the light source 12 and/or in association with the goggles 22, namely as one or more lenses 20ₐ. As used herein, the term "lens" is intended to include any light modifying element, e.g., filter elements, such as a filter excluding light of particular wavelengths and/or polarizing elements. More particularly, the lenses 20ₐ, 20_{b} of the goggles 22 may be composite, i.e., may be composed of multiple lenses/filters/polarizers to achieve a desired transmissibility to the reflected light 18. Having passed through the lenses 20ₐ, 20_{b} of the goggles 22, the observed light 19 can then be seen by the eye E of the person P. The foregoing apparatus and associated method therefore presents a system 5 whereby skin conditions may be visualized by utilizing a simple hand held light source 12 similar in size to a flashlight that produces specific and selectable wavelengths and/or polarization characteristics. These selected wavelength and/or polarizing characteristics of the light source are then coupled with selected filtering or polarization lenses associated with the goggles 22 in order to highlight specific skin conditions. While eyeglass-type goggles 22 with fixed lenses 20a, 20b are shown in FIG. 1, goggles 22 utilizing frames for receiving clip-on or screw-on lenses may be employed. Stacking of lenses, if desired, may be accomplished by using conventional clip-on or screw-on arrangements that are known in the fields of optometry and photography, e.g., as illustrated by the various types of clip-on sunglasses, or clip-on or threaded filters for use on cameras. Similarly, while fixed lenses are shown, lenses that are rotatably mounted to the frame of the goggles 22 or that may be affixed to the goggles 22 in a plurality of orientations is within the scope of the present invention and would be useful for affixing polarizing lenses to the goggles 22. Exemplary means for selecting and positioning lenses 20a, 20b singly or in combination are shown in FIGS. 8-12. A system 5 in accordance with the present invention therefore allows a user to self-visualize and evaluate skin conditions in order to select and apply skin products based upon changing needs and/or to visualize skin improvement resulting from successful therapy with these products.

The system 5 comprehends a single-use device e.g., a pair of goggles with a single type of lenses 20a, 20b and a light 12 having a single illuminating light output, or may use multiple light sources/filters and goggle combinations. A plurality of different lenses/filters may be provided to cooperate with a single pair of goggles 22 and a single light 12, or a set of different goggles 22 and/or set of different light sources 12 may be utilized.

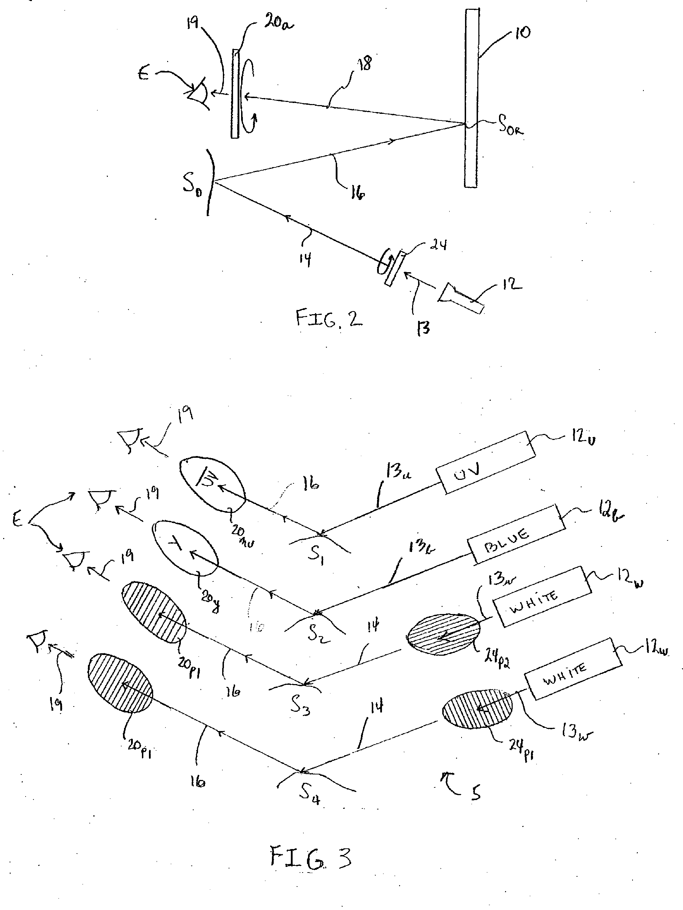

Various selectable components and combinations of lenses 20 and lights 12 for the system 5 are illustrated in figure 3 wherein light sources 12ᵤ, 12_{b}, 12_{w} radiate light in a specific range of wavelengths 13ᵤ, 13_{b}, 13_{w}. In the case of the white light sources 12_{w}, the emitted white light 13_{w} is passed through polarizing elements, either 24ₚ₁ or 24ₚ₂ before it is reflected off the surface to be viewed, e.g., S₃ or S₄. The UV light source 12u is not filtered or polarized, such that the light 13ᵤ emitted therefrom reflects from surface S₁ to produce resultant reflected light 16. Similarly the blue light 13b from the blue light source 12_{b} is not polarized or filtered prior to reflecting off surface S₂ to produce reflected light 16. Having reflected off the surfaces S₁, S₂, S₃, S₄, the reflected light 16 may be further processed by a polarizer set at a specific orientation, e.g., 20ₚ₁, a light filter, e.g., a yellow light filter 20_{y}, or a UV light filter 20ₙᵤ. Having passed through the filters and/or polarizers 20 which are associated with the goggles 22, the observed light 19 then enters the eye E of the person visualizing the skin.

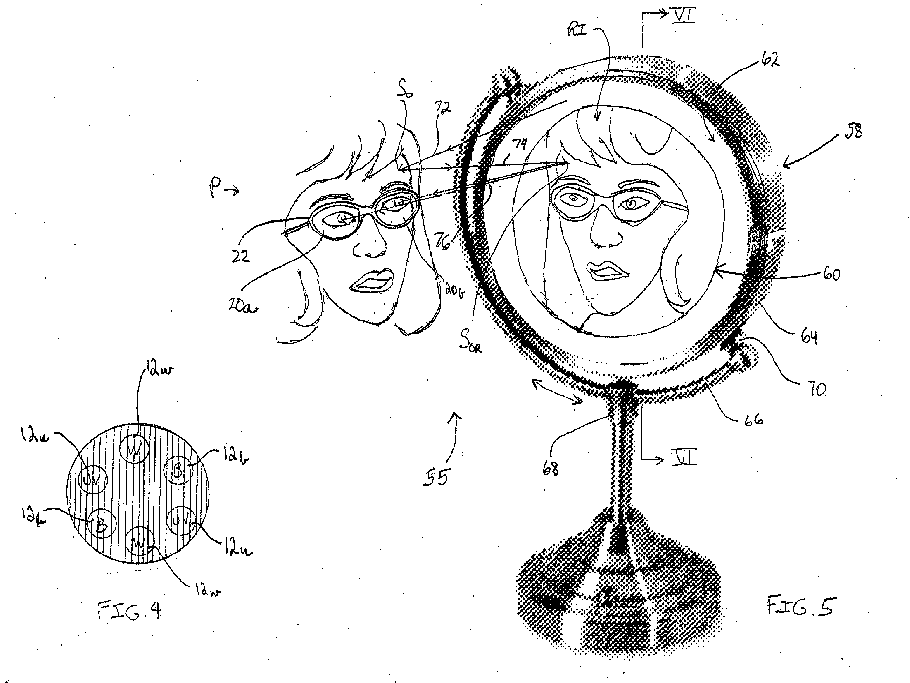

FIG. 4 shows a configuration of multiple light sources (bulbs, LEDs filtered light emitters) 12_{b}, 12_{w}, 12ᵤ that could be utilized in light 12. The light 12 may have multiple emitters for emitting specific wavelengths of light upon selection via a switch disposed on the surface of the light 12, for viewing specific skin features. The system 5 may be used for enhanced viewing of several different skin conditions/states. The following examples are illustrative.

For viewing skin wrinkles, the light 12 may include a white light source, such as a tungsten lamp, white LED, fluorescent bulb, or metal halide light. Illuminating light is filtered through a polarizing lens 24 disposed on the output of the light 12. The light 12 may be held by the user and shone onto the face (or body) to be analyzed in front of a normal mirror. The user wears a set of goggles 22 that include polarizing filter lenses 20ₐ, 20_{b} oriented in the same polarization direction as the polarizing lens 24. This configuration of the system 5 enhances the visualization of surface texture and wrinkles of the skin, while obscuring the skin's sub-surface characteristics that can obscure surface information.

To view skin inflammation and sub-surface blood vessels, the light 12 includes a white light source, such as a tungsten lamp, white LED, fluorescent bulb, or metal halide light. The illuminating light is filtered through a polarizing lens 24 on the output of the light 12. The light 12 may be held by the user and shone onto the face (or body) to be analyzed in front of a normal mirror. The orientation of the polarization filter 24 is perpendicular to polarizing filter lenses 20ₐ, 20_{b} of the goggles 22. In this configuration, the system 5 eliminates the visualization of the surface texture and wrinkles of the skin, while enhancing the skin's sub-surface characteristics such as the redness and vasculature under the skin's surface.

Acne related conditions, i.e., horn and bacteria may be viewed with a light 12 emitting blue light (380-420nm), which can be produced by LED's, or white light from a tungsten bulb or metal halide or blue fluorescent source, that is shone onto the face of the user. The user wears goggles 22 with yellow lenses 20ₐ, 20_{b}, which block the incident blue light and allows for the visualization of porphyrin fluorescence (red color) showing where bacteria is active in producing porphyrins from sebum and the location of individual comedones expressing horn (epidermal cells with sebaceous material) which fluoresces white. Using this configuration of the system 5, areas of acne activity prior to inflammatory breakout may be visualized.

The system 5 may be utilized to view UV photodamage, collagen and elastin florescence and pigmentation by using a light 12 emitting UV light (320-400nm). UV light can be produced by a fluorescent black light bulb, a filtered tungsten lamp, a filtered zenon lamp, a UV LED, or filtered metal halide source. The UV light is shone onto the face or body of the user to fluoresce the collagen and elastin in the skin. The level of fluorescence is dependent on the amount of photo damage and skin age of the individual. The amount of pigment and distribution of pigment is highlighted by the underlying collagen and elastin fluorescence and indicates the extent of photodamage to the individual's skin. The user should wear goggles 22 with UV filtering lenses 20ₐ, 20_{b} that block UV radiation to protect the eyes from the potentially harmful UV.

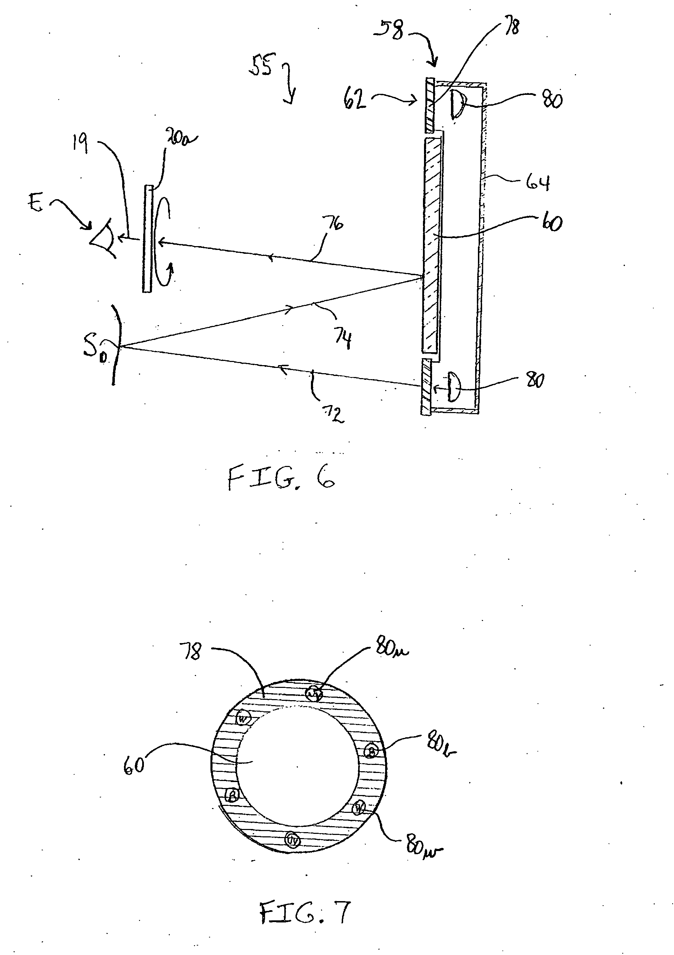

FIG. 5 shows an alternative embodiment of the system 55 in accordance with the present invention utilizing an illuminating mirror 58. The illuminating mirror 58 has a mirror portion 60, which may be a conventional reflective mirror to provide a reflected image RI. The mirror portion 60 is surrounded by a ring-shaped light source 62 which may be in the form of a plurality of individual illuminating elements/ lights 80 that are disposed about the periphery of the mirror portion 60. The lights 80 are mounted in housing 64 and the housing may accommodate a diffuser or polarizing ring 78 as shall be described below. The housing 64 is pivotally mounted on mounting ring 66 at pivots 70 to permit repositioning of the mirror along the axis established between the pivots 70. In addition to pivoting, the illuminating mirror 58 can be rotated by sliding the mounting ring 66 through the support post 68 to establish a particular angular orientation of the light source 62. This ability to rotate the illuminating mirror on the mounting ring 66 is useful in the instance where the light source 62 includes a polarizing element disposed thereover to polarize the light 72 emanating from the light source 62. In this manner, the direction of the polarizing element can be changed to conduct various skin examinations, i.e., to position the polarizing element perpendicular to or parallel to the orientation of polarizer elements associated with the goggles 22 in a similar fashion as the polarizing elements 24ₚ₁ and 24ₚ₂ may be orientated relative to polarizing elements 20ₚ of the prior embodiment described above. As in the prior embodiment, light 72 projected from the light source 62 impinges upon a surface S₀ on the face of the person P and is reflected therefrom 74 to a corresponding point S₀ᵣ on the reflected image RI appearing in the mirror. The light is then reflected from surface S₀ᵣ towards the lenses 20ₐ, 20_{b} of the goggles 22 where it is then converted into the observed light 19 due to its passage through the lens elements 20ₐ, 20_{b}.

FIGS. 6 and 7 show that lights 80 within the light source 62 may be a plurality of independent bulbs or LEDs which generate specific wavelengths of light, viz., UV light 80ᵤ, blue light 80_{b}, and white light 80_{w}. As noted above, various combinations of lenses, filters and polarizers can be used in conjunction with the lights 80 to produce a desired illuminating light 72.

FIGS. 8 and 9 show goggles 82 having threaded flanges 84 for receiving one or more nested lenses 86a, 86b, which may be filters or polarizers as described above.

FIG. 10 shows conventional clip-on style lenses 88 with hooks for grasping the frame of a pair of goggles 22.

FIGS. 11 and 12 show goggles 92 with a slotted frame 94 for receiving lens inserts 96a, 96b. In the embodiment shown, the lens inserts 96a, 96b are rotatable via tab 98, e.g., to serve as adjustable polarizers.

## Claims

1. Apparatus for aiding a person illuminated by a light source to view their skin in a mirror, the light source emanating light that impinges upon the skin of the person, causing light to be reflected from the skin and causing the skin to emit light, the reflected and emitted light impinging on the mirror and further reflecting from the mirror to the eye of the person, the emanation of light from the light source and subsequent reflections and emanation from the skin and the mirror to the eye defining a path of light energy from the light source to the eye, comprising:
a light modification element other than a magnifying lens interposed in the path of light energy between the light source and the eye of the person viewing the skin, said light modification element enhancing the visualization of at least one attribute of the skin.

2. The apparatus of Claim 1, wherein the light modification element is positioned between the mirror and the eye.

3. The apparatus of Claim 2, wherein the light modification element is a filter.

4. The apparatus of Claim 2, wherein said light modification element is a polarizer.

5. The apparatus of Claim 2, further including a second light modification element interposed into the path of light energy, said second light modification element being positioned between the light source and the person.

6. The apparatus of Claim 5, wherein the light source is a source of at least one of white light, blue light and UV light, the light modification element is at least one of a polarizer at a selected angular orientation and a light filter and said second light modification element is at least one of a polarizer at the same orientation as the light modification element, a polarizer at a different orientation as the light modification element, a yellow light filter and a UV filter.

7. The apparatus of Claim 2, further including means for holding the light modification element.

8. The apparatus of Claim 7, wherein said holding means allows said light modification element to be rotated relative thereto.

9. The apparatus of Claim 7, wherein said holding means allows said light modification element to be removable from the path of light energy.

10. The apparatus of Claim 7, wherein said holding means allows a plurality of light modification elements to be held in said path of light energy.

11. The apparatus of Claim 1, wherein said light modification element is positioned in the path of light energy between the light source and the person.

12. The apparatus of Claim 11, wherein said light modification element is a filter.

13. The apparatus of Claim 11, wherein said light modification element is a polarizer.

14. The apparatus of Claim 11, wherein said light modification element is removable.

15. The apparatus of Claim 11, wherein said light modification element is rotatable relative to the light source.

16. A kit for enhanced self-inspection in a mirror, comprising:
a light source;
a light modification element other than a magnifying lens through which a mirror image of a person may be viewed, said light modification element enhancing the visualization of at least one attribute of the skin.

17. The kit of claim 16, wherein said light source emits a plurality of wavelengths of light and further including holding means for holding said light modification element in the line of sight of the person viewing the mirror image, said light modification element being at least one of a light filter and a polarizer, further including a second light modification element being at least one of a filter and a polarizer, said second light modification element, being rotatable, attachable and removable relative to said light source.

18. The kit of Claim 16, further including the mirror, said light source being disposed around a periphery of said mirror and a second light modification element, being at least one of a filter and a polarizer, at least one of said mirror, said polarizer and said light source being rotatable to assume a plurality of orientations, said first light modification element being a lens element in a goggle wearable by the person.

19. The kit of Claim 18, wherein said light source selectively emits light of a plurality of wavelengths.

20. A method for a person conducting self-examination of the skin in a mirror, comprising:
(A) illuminating the skin with a light source emanating light of a selected wavelength and state of polarization/nonpolarization to create a path of light energy from the light source to the eye of the person ; and
(B) positioning a light modification element other than a magnifying lens in the path of light to enhance the person's view of themselves in the mirror, such that the perceptibility of an attribute of their skin is visually enhanced over that otherwise viewable in the mirror without the light modification element.

21. The method of Claim 20, wherein the polarization of the light source is the same as the polarization of the light modification element to enhance the visualization of superficial skin surface features.

22. The method of Claim 20, applied to the self-examination of sub-surface skin features such as vascular components and pigment, wherein the polarization of the light source is 90 degrees relative to the polarization of the light modification element to enhance viewing of the skin's subsurface features and chromophores.

23. The method of Claim 20, applied to the self-examination of sub-surface photodamage, wherein the light source emits ultraviolet or near blue (320-440 nm) light and the light modification element blocks transmission of these same wavelengths and only passes wavelengths greater than the illumination wavelengths to visualize collagen fluorescence and pigment blockage of collagen fluorescence.

24. The method of Claim 20, applied to the self-examination of fluorescent elements associated with acne bacterium and metabolism and buildup of keratin plugs on the skin, wherein the illumination wavelengths on the skin are in the wavelength region of 385 to 420, and the light modification element is a yellow filter.
